# EUROPEAN PATENT APPLICATION

(11) **EP 1 783 201 A1**
(43) Date of publication of application: **09.05.2007**
(21) Application number: 05110336.4
(22) Date of filing: 04.11.2005
(51) Int. Cl.: C11D 3/20, C07D 305/12, C07C 59/76

(54) **Surfactant precursor**

(71) Applicant: YKI, Ytkemiska Institutet AB, 11486 Stockholm (SE)
(72) Inventor: Andersson, Martin, SE-115 30 Stockholm (SE); Kronberg, Bengt, SE-115 30 Stockholm (SE)
(74) Representative: Mattsson, Niklas

(57) **Abstract**

Use, as a surfactant precursor, of a compound having the general formula I 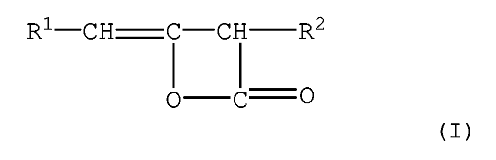 wherein at least one of R¹ and R² is a hydrophobic moiety. A dispersion comprising a surfactant obtained by activation of said surfactant precursor. A method for breaking a dispersion comprising a surfactant obtained by activation of said surfactant precursor. A microemulsion comprising, as a surfactant, a compound obtained by activation of said surfactant precursor. A method for reducing the surface tension between a liquid and another phase, using said surfactant precursor. Various applications making use of the surfactant properties of the surfactant obtained by activation of said surfactant precursor.

## Description

### Technical field of the invention

The present invention relates to use, as a surfactant precursor, of a compound having the general formula I 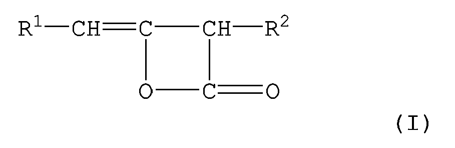 wherein at least one of R¹ and R² is a hydrophobic moiety, to a dispersion comprising a surfactant obtained by activation of said surfactant precursor, to methods for preparing and breaking a dispersion comprising a surfactant obtained by activation of said surfactant precursor, to a microemulsion comprising, as a surfactant, a compound obtained by activation of said surfactant precursor, to a method for reducing the surface tension between a liquid and another phase, using said surfactant precursor, and to various applications making use of the surfactant properties of the surfactant obtained by activation of said surfactant precursor.

### Background art

So-called cleavable surfactants have been known for several years and the different classes of such surfactants have been nicely reviewed by Stjerndahl et al. in "Cleavable Surfactants, Novel Surfactants - Preparation, Applications, and Biodegradability", 2 ed., Holmberg ed., Marcel Dekker, Inc., USA, 2003. A main reason for the development of cleavable surfactants has been environmental concern and a desire for biodegradable surfactants. The main types of cleavable surfactants known today are:
1) Surfactants labile at acidic conditions
2) Surfactants labile at alkaline conditions
3) Light sensitive surfactants
4) Surfactants that degrade in contact with specific chemicals
5) Thermolabile surfactants

According to Stjerndahl, most cleavable surfactants contain a hydrolysable bond and thus belong to type 1 or 2. For these surfactants, a change of pH is needed to initiate cleaving of the surfactant. The degradation product is often a soap or a long-chain alcohol, of which at least the former is clearly surface active. Thus, the cleaving of these compounds does not automatically imply that surface activity is lost.

In the case of light sensitive surfactants (type 3), the surfactant has to be exposed to light for a certain amount of time to obtain the desired cleaving.

Surfactants that decompose in contact with specific chemicals (type 4), e.g. ozone cleavable surfactants, are used in specific applications only.

For the group of known thermolabile surfactants (type 5), the decomposition rate is intended to be controlled by regulation of the temperature.

Hayashi et al. (JAOCS 62(3):555-557 (1985)) report preparation of amine oxide surfactants by oxidation of 2-alkoxy-N,N-dimethylethylamines with hydrogen peroxide. The 2-alkoxy-N,N-dimethylethylamine N-oxide surfactants formed were good foam stabilizers and stable up to 100 °C, but decomposed rapidly to vinyl ethers at 150 °C. Hence, the decomposition temperature of these surfactants is not compatible with use in aqueous compositions.

GB 923,449 discloses that unsaturated polymerisable compounds can be advantageously polymerised in aqueous medium and in the presence of dispersing agents and activators by using as a dispersing agent a salt of a partial ester of an aliphatic polycarboxylic acid with one or more alcohols having 3 to 20 carbon atoms and heating the resultant polymer emulsion at temperatures between 60 and 200 °C. However, these dispersing agents have the drawback of being intrinsically sensitive to alkaline and acidic conditions, causing premature degradation of the surfactant under such conditions.

Existing cleavable surfactants and dispersing agents of types 1-4 are either adapted for speciality applications only (being light sensitive or dependent on a specific substance) or cannot be utilised and cleaved at substantially constant pH conditions. Furthermore, cleaving does not always result in loss of surface activity. Existing thermolabile surfactants (type 5) are not suitable in water based applications and/or under alkaline or acidic conditions.

AKD, alkyl ketene dimers, is a group of molecules with a wide range of industrial applications. For example, AKD is used as reactive size in paper manufacture. AKD reacts with hydroxyl groups of the cellulose and provides the paper with a hydrophobic surface. This is described in Roberts, J. C., "Neutral and Alkaline Sizing", Paper Chemistry, Roberts, J. C., Ed., Chapman and Hall: London, 1996, pp 140-160.

In the use of AKD in paper sizing, hydrolysis is considered an unwanted side-reaction having a detrimental effect on the performance of the sizing process. This has been described by Joseph Marton in Tappi Journal, 73(11), 139-143, November 1990.

### Summary of the invention

An object of the present invention is to provide a compound for use as a surfactant precursor, which can be used to provide a deactivable thermolabile surfactant, the decomposition rate of which can be controlled within wide limits by altering the temperature in a range starting already at or below room temperature and with no need to go to extremely high temperatures (e.g. over the boiling point of water) for fast decomposition.

Another object of the invention is to provide a surfactant precursor which upon activation provides a surfactant that is not susceptible to premature and/or thermally uncontrollable decomposition.

Yet another object of the invention is to provide a surfactant precursor which upon activation provides a surfactant which may be designed to give only non-toxic products upon decomposition.

A further object of the invention is to provide a dispersion comprising a thermolabile surfactant, said dispersion having a thermally controllable delay time before breaking and a thermally controllable breaking rate, wherein said surfactant is obtained by activation of a surfactant precursor according to the invention.

A further object of the invention is to provide a method for breaking a dispersion.

In industrial use of cleavable surfactants, it is of material importance that the surfactants can be stored before use without any considerable premature surfactant degradation. It is therefore an object of the present invention to provide the use of a thermally stable surfactant precursor, which can quickly be activated to a decomposable surfactant on-site or just before use.

The above-mentioned objects as well as other objects of the invention, which should be apparent to a person skilled in the art after having studied the description below, are accomplished by the use of a surfactant precursor comprising at least one hydrophobic moiety.

Thus, the invention provides use, as a surfactant precursor, of a compound having the general formula I 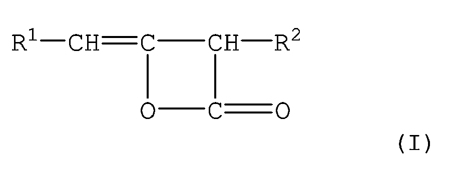 wherein at least one of R¹ and R² is a hydrophobic moiety.

In an embodiment of the invention R¹ is a hydrophobic moiety.

In an embodiment of the invention R² is a hydrophobic moiety.

In an embodiment of the invention R¹ and R² are both hydrophobic moieties.

In an embodiment of the invention R¹ and R² are both hydrophobic moieties of the same type.

A typical characteristic of a surfactant precursor according to the invention is that it does not degrade or decompose rapidly at room temperature. Thus, a thermolabile surfactant can be stored in the form of a thermally stable surfactant precursor according to the present invention, which can quickly be activated to said surfactant on-site or just before use. The surfactant precursor of the present invention may be activated (i.e. transformed to the surfactant) by hydrolysis, optionally followed by an adjustment of pH. The concept of hydrolysis is well known to the skilled man. Activation of the surfactant precursor of the present invention to the corresponding surfactant can be achieved by hydrolysis according to the general reaction scheme below.

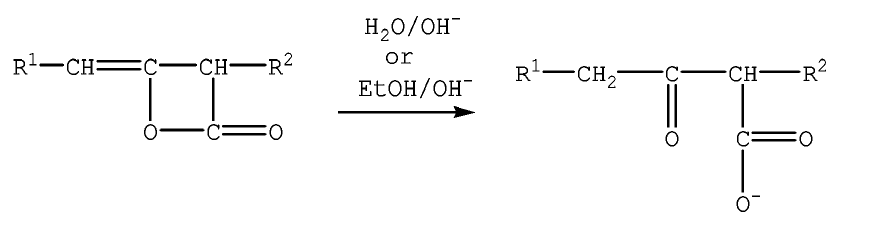

The surfactant precursor of the present invention can be synthesized by chlorination of fatty acids followed by dimerisation of the formed fatty acid chlorides. Different fatty acids can be used, resulting in a surfactant precursor compound comprising the corresponding fatty acid residues, which then correspond to R¹ and R². Hence, a wide spectrum of different surfactant precursor compounds can be obtained by use of different fatty acids and combinations thereof. Fatty acids that can be used in the synthesis of a surfactant precursor according to the present invention may comprise, but are not limited to, caproic, caprylic, capric, lauric, myristic, palmitic, palmitoleic, stearic, isostearic, oleic, linoleic, linolenic, arachidic, gadoleic, behenic and erucic acids and combinations thereof.

The fatty acids used in the synthesis of the surfactant precursor may be derived from naturally occurring or synthetically produced fats or oils. Non-limiting examples of useful fats and oils are soybean oil, rapeseed oil, oiticica oil, tung oil, castor oil, tall oil, butterfat, lard, tallow, herring oil, menhaden oil, sardine oil, whale oil, olive oil, palm oil, safflower oil, sesame oil, sunflower oil, linseed oil, babassu oil, coconut oil, palm kernel oil, corn oil, cottonseed oil and groundnut oil.

The invention further provides use of a compound as defined by the general formula I as a precursor for a surfactant having the general formula II 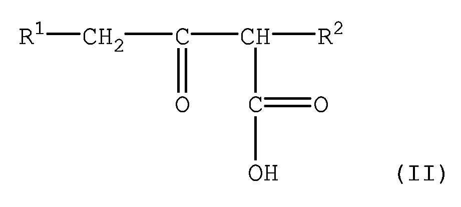 wherein at least one of R¹ and R² is a hydrophobic moiety, or as a precursor for a salt of said surfactant.

The surfactant obtained upon activation of a surfactant precursor of the present invention has been found to be susceptible to temperature controlled decomposition into CO₂, HCO₃⁻ or CO₃²⁻ (depending on pH) and an oil-like (if liquid), hydrophobic residue, thereby reducing its surface activity. Furthermore, said surfactant is stable to premature and/or thermally uncontrollable degradation over a wide pH range and is thus protected from unintentional decomposition at alkaline, neutral or acidic conditions (generally at pH ≥ p*Kₐ*). Depending on present pH conditions, a salt of the described surfactant may be active.

A further characteristic of a surfactant obtained upon activation of a surfactant precursor of the present invention is that its rate of decomposition, and thus its rate of inactivation, can be effectively controlled by temperature at substantially constant pH conditions. Its rate of decomposition generally increases with increasing temperature.

It is well known to the skilled man that a wide selection of hydrophobic groups exists and that their detailed structures are not always of critical importance. As non-limiting examples, the hydrophobic moiety or moieties R¹ and/or R² in formulas I or II may independently be a straight-chain, branched-chain or cyclic, saturated or unsaturated, optionally substituted, aliphatic group; an optionally substituted aromatic group; an optionally substituted hydrophobic polyoxyalkylene group, such as an optionally substituted polyoxypropylene group; an optionally substituted perfluoroalkyl group; an optionally substituted polysiloxane group; a lignin or rosin derivative; or a combination thereof.

As used herein, the term "substituted", in relation to the hydrophobic moiety or moieties, relates to the substitution of an organic group with any substituents not changing the hydrophobic nature of said moiety or the amphiphilic nature of a surfactant obtained upon activation of the compound of the invention.

The hydrophobic moiety or moieties is/are preferably independently selected from a straight-chain, branched-chain or cyclic, saturated or unsaturated, optionally substituted, aliphatic group; an optionally substituted aromatic group; and any combination thereof. More preferably, the hydrophobic moiety or moieties is/are independently selected from a straight-chain or branched-chain, saturated or unsaturated, optionally substituted, C₄-C₃₀ alkyl, or C₄-C₂₂ alkyl.

Non-limiting examples of hydrophobic groups R¹ and R² that may independently be present in a surfactant precursor as defined by the invention: -(CH₂)₃-CH₃; -(CH₂)₄-CH₃; -(CH₂)₅-CH₃; -(CH₂)₆-CH₃; -(CH₂)₇-CH₃; -(CH₂)₈-CH₃; -(CH₂)₉-CH₃; -(CH₂)₁₀-CH₃; -(CH₂)₁₁-CH₃; -(CH₂)₁₂-CH₃; -(CH₂)₁₃-CH₃; -(CH₂)₁₄-CH₃; -(CH₂)₁₅-CH₃ ; -(CH₂)₁₆-CH₃; -(CH₂)₁₇-CH₃; -(CH₂)₁₈-CH₃; -(CH₂)₁₉-CH₃ ; - (CH₂)₆-CH=CH-(CH₂)₅-CH₃; - (CH₂)₁₃-CH(CH₃)-CH₃ ; - (CH₂)₆-CH=CH-(CH₂)₇-CH₃; -(CH₂)₆-CH=CH-CH₂-CH=CH-(CH₂)₄-CH₃ ; - (CH₂)₆-CH=CH-(CH₂)₉-CH₃; -(CH₂)₁₀-CH=CH-(CH₂)₇-CH₃ ; - (CH₂)₆-CH=CH-CH₂-CH=CH-CH₂-CH=CH-CH₂-CH₃ ; - (CH₂-CH(CH₃)-O)₄-H ; -CH₂-(O-CH(CH₃)-CH₂)₄-OH; -(CF₂)₆-CF₃ ;

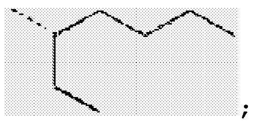

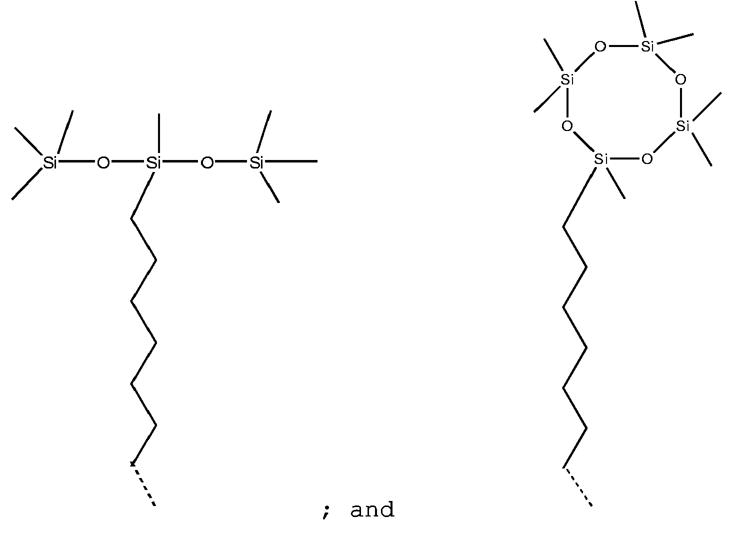

In some preferred embodiments of the invention, any hydrophobic moieties R¹ and R² present in the surfactant precursor are independently selected from: -(CH₂)₃-CH₃; -(CH₂)₅-CH₃; -(CH₂)₇-CH₃; -(CH₂)₉-CH₃; -(CH₂)₁₁-CH₃; -(CH2)₁₃-CH₃; -(CH₂)₁₅-CH₃; -(CH₂)₁₇-CH₃; -(CH₂)₁₉-CH₃; - (CH₂)₆-CH=CH-(CH₂)₅-CH₃; - (CH₂) ₁₃-CH (CH₃) -CH₃; - (CH₂)₆-CH=CH-(CH₂)₇-CH₃; -(CH₂) ₆-CH=CH-CH₂-CH=CH- (CH₂) ₄-CH₃; - (CH₂) ₆-CH=CH-CH₂-CH=CH-CH₂-CH=CH-CH₂-CH₃; - (CH₂) ₆-CH=CH- (CH₂)₉-CH₃; and - (CH₂)₁₀-CH=CH- (CH₂) ₇-CH₃.

In further preferred embodiments of the invention, any hydrophobic moieties R¹ and R² present in the surfactant precursor are independently selected from: -(CH₂)₉-CH₃; -(CH₂)₁₁-CH₃; -(CH₂)₁₃CH₃; -(CH₂)₁₃-CH₃; -(CH₂)₁₉-CH₃; -(CH₂)₁₃-CH(CH₃)-CH₃; -(CH₂)₆-CH=CH-(CH₂)₇-CH₃; - (CH₂)₆-CH=CH-CH₂-CH=CH-(CH₂)₄-CH₃; - (CH₂)₆-CH=CH-CH₂-CH=CH-CH₂-CH=CH-CH₂-CH₃; -(CH₂)₆-CH=CH-(CH₂)₉-CH₃; and - (CH₂)₁₀-CH=CH- (CH₂)₇-CH₃.

In yet further preferred embodiments of the invention, any hydrophobic moieties R¹ and R² present in the surfactant precursor are independently selected from - (CH₂)₁₃-CH₃ and - (CH₂)₁₅-CH₃.

The groups listed above are common, cheap, safe and well-functioning hydrophobic groups.

In specific but non-limiting embodiments of the invention, the hydrophobic moieties R¹ and R² are present in a surfactant precursor in combinations as disclosed in Table 1.

**Table 1**

| Hydrophobic substituents in compounds for use as surfactant precursors in accordance with the present invention | | |
|---|---|---|
| Cpd # | R¹ | R² |
| 1 | -(CH₂)₁₁-CH₃ | -(CH₂) ₁₁-CH₃ |
| 2 | - (CH₂) ₁₁-CH₃ | - (CH₂) ₁₃-CH₃ |
| 3 | -(CH₂)₁₁-CH₃ | -(CH₂)₁₅-CH₃ |
| 4 | - (CH₂)₁₁-CH₃ | - (CH₂) ₆-CH=CH- (CH₂) ₇-CH₃ |
| 5 | -(CH₂)₁₁-CH₃ | - (CH₂)₆-CH=CH-CH₂- CH=CH- (CH₂)₄-CH₃ |
| 6 | -(CH₂)₁₁-CH₃ | - (CH₂)₆-CH=CH-CH₂- CH=CH-CH₂-CH=CH-CH₂-CH₃ |
| 7 | -(CH₂)₁₃-CH₃ | -(CH₂)₁₁-CH₃ |
| 8 | -(CH₂)₁₃-CH₃ | -(CH₂)₁₃-CH₃ |
| 9 | -(CH₂)₁₃-CH₃ | - (CH₂)₁₅-CH₃ |
| 10 | -(CH₂)₁₃-CH₃ | -(CH₂)₆-CH=CH-(CH₂)₇-CH₃ |
| 11 | -(CH₂)₁₃-CH₃ | - (CH₂)₆-CH=CH-CH₂- CH=CH- (CH₂)₄-CH₃ |
| 12 | -(CH₂) ₁₃-CH₃ | - (CH₂)₆-CH=CH-CH₂- CH=CH-CH₂-CH=CH-CH₂-CH₃ |
| 13 | -(CH₂)₁₅-CH₃ | -(CH₂)₁₁-CH₃ |
| 14 | -(CH₂)₁₅-CH₃ | -(CH₂)₁₃-CH₃ |
| 15 | -(CH₂)₁₅-CH₃ | -(CH₂)₁₅-CH₃ |
| 16 | -(CH₂)₁₅-CH₃ | -(CH₂)₆-CH=CH- (CH₂)₇-CH₃ |
| 17 | -(CH₂)₁₅-CH₃ | - (CH₂)₆-CH=CH-CH₂- CH=CH- (CH₂)₄-CH₃ |
| 18 | -(CH₂)₁₅-CH₃ | - (CH₂)₆-CH=CH-CH₂- CH=CH-CH₂-CH=CH-CH₂-CH₃ |
| 19 | -(CH₂)₆-CH=CH-(CH₂)₇-CH₃ | - (CH₂) ₁₁-CH₃ |
| 20 | -(CH₂)₆-CH=CH-(CH₂)₇-CH₃ | - (CH₂) ₁₃-CH₃ |
| 21 | -(CH₂)₆-CH=CH-(CH₂)₇-CH₃ | - (CH₂) ₁₅-CH₃ |
| 22 | -(CH₂)₆-CH=CH-(CH₂)₇-CH₃ | -(CH₂)₆-CH=CH-(CH₂)₇-CH₃ |
| 23 | -(CH₂)₆-CH=CH-(CH₂)₇-CH₃ | -(CH₂)₆-CH=CH-CH₂- CH=CH- (CH₂) ₄-CH₃ |
| 24 | -(CH₂)₆-CH=CH-(CH₂)₇-CH₃ | -(CH₂)₆-CH=CH-CH₂- CH=CH-CH₂-CH=CH-CH₂-CH₃ |
| 25 | - (CH₂) ₆-CH=CH-CH₂- CH=CH- (CH₂)₄-CH₃ | - (CH₂) ₁₁-CH₃ |
| 26 | - (CH₂)₆-CH=CH-CH₂- CH=CH-(CH₂)₄-CH₃ | - (CH₂)₁₃-CH₃ |
| 27 | - (CH₂)₆-CH=CH-CH₂- CH=CH-(CH₂)₄-CH₃ | - (CH₂)₁₅-CH₃ |
| 28 | - (CH₂) ₆-CH=CH-CH₂- CH=CH- (CH₂) ₄-CH₃ | - (CH₂) ₆-CH=CH- (CH₂) ₇-CH₃ |
| 29 | - (CH₂) ₆-CH=CH-CH₂- CH=CH- (CH₂) ₄-CH₃ | - (CH₂) ₆-CH=CH-CH₂- CH=CH- (CH₂) ₄-CH₃ |
| 30 | - (CH₂) ₆-CH=CH-CH₂- CH=CH- (CH₂) ₄-CH₃ | - (CH₂) ₆-CH=CH-CH₂- CH=CH-CH₂-CH=CH-CH₂-CH₃ |
| 31 | - (CH₂)₆-CH=CH-CH₂- CH=CH-CH₂-CH=CH-CH₂-CH₃ | - (CH₂)₁₁-CH₃ |
| 32 | - (CH₂)₆-CH=CH-CH₂- CH=CH-CH₂-CH=CH-CH₂-CH₃ | - (CH₂)₁₃-CH₃ |
| 33 | - (CH₂)₆-CH=CH-CH₂- CH=CH-CH₂-CH=CH-CH₂-CH₃ | - (CH₂)₁₅-CH₃ |
| 34 | - (CH₂)₆-CH=CH-CH₂- CH=CH-CH₂-CH=CH-CH₂-CH₃ | - (CH₂)₆-CH=CH-(CH₂)₇-CH₃ |
| 35 | - (CH₂)₆-CH=CH-CH₂- CH=CH-CH₂-CH=CH-CH₂-CH₃ | - (CH₂)₆-CH=CH-CH₂- CH=CH-(CH₂)₄-CH₃ |
| 36 | - (CH₂)₆-CH=CH-CH₂- CH=CH-CH₂-CH=CH-CH₂-CH₃ | - (CH₂)₆-CH=CH-CH₂- CH=CH-CH₂-CH=CH-CH₂-CH₃ |

As explained above, the skilled person is able to select from the multitude of known hydrophobic groups in order to obtain the compounds for use as surfactant precursors in accordance with the present invention, and hence surfactants obtained upon activation thereof. Known parameters of importance to consider when selecting hydrophobic moieties for the compounds for use as surfactants are, among others, the hydrophilic-lipophilic balance (HLB) and the Krafft temperature of the surfactant. The determination of these parameters is within the abilities of a person of skill in the art, but the two concepts are nevertheless outlined below for completeness.

The so-called hydrophilic-lipophilic balance (HLB) of a given surfactant is a well-known characteristic, tabulated for commercial surfactants and used extensively for selection of a suitable surfactant for a given application. A high HLB number means that the surfactant in question has a high affinity for the water-phase if present in a water + oil system. If an emulsion is prepared from such a system, the result is normally an oil-in-water emulsion. On the other hand, if the HLB number is low, the surfactant has a high affinity for the oil phase, and water-in-oil emulsions are normally formed. The HLB number of a surfactant is influenced by the chemical structure of both hydrophilic and hydrophobic groups of the surfactant. Within the surfactant class provided by the surfactant precursor of the present invention, the HLB parameter can be varied mainly through selection of hydrophobic group. As an example, those surfactants having a shorter carbon chain as hydrophobic group(s) will have a higher HLB number (will have a higher affinity for water) than those having longer carbon chains.

The Krafft temperature is the temperature at which the solubility in water increases very dramatically. For a given application using a surfactant provided by a surfactant precursor according to the invention, it is important to consider the Krafft temperature of the surfactant, so as to assure dissolution of the surfactant at the temperature in question. A well-known way of decreasing the Krafft temperature of a surfactant is the use of one or several double bonds in the hydrophobic group(s), creating a bent carbon chain which in turn renders packing conditions in the solid state less favourable. As an example, those surfactant precursors in the above listing comprising at least one double bond should provide surfactants having a lower Krafft temperature than the corresponding compounds without the double bond. Another well-known way of lowering the Krafft temperature of a surfactant is to introduce branching in the hydrophobic group.

A compound obtained upon activation of a surfactant precursor of the present invention acts as an anionic surfactant (i.e. in its salt form) at pH ≥ p*Kₐ*. The p*Kₐ* of compounds of the present invention may vary widely, e.g. as a result of the choice of hydrophobic moieties.

The skilled man is able to choose suitable temperatures (e.g. in the range from 0 to 100 °C) for employing a compound obtained upon activation of a surfactant precursor of the present invention.

The surfactant obtained upon activation of a surfactant precursor of the present invention may be a dispersing agent.

The objects of the present invention are also accomplished by a dispersion comprising solid particles, liquid droplets or gas bubbles dispersed, as an internal phase, in a fluid, as an external phase, by means of a surfactant, as a dispersing agent, wherein said surfactant is obtained by activation of a surfactant precursor as defined above.

Said surfactant may be active in any kind of dispersion, the preparation of which can be accomplished according to procedures well known to the skilled man (such as agitation, shearing or spraying). As is also well known to the skilled man, a dispersion may be an aerosol, a colloid, an emulsion, a foam, a gel, a sol or a suspension. The external phase of such a dispersion may be an aqueous phase as well as an oil phase or air. The skilled man is able to routinely examine the decomposition rate, as manifested by delayed dispersion breaking and/or the breaking rate at different temperatures in such a dispersion or as manifested by a decrease in foamability (cf. Example 3 below). Accordingly, the skilled man is able to choose suitable temperatures (e.g. in the range from 0 to 100 °C) for employing the dispersion. The rate of decomposition can be effectively controlled by temperature at substantially constant pH conditions. Its rate of decomposition is generally increasing with increasing temperature.

The objects of the present invention are further met by a method for preparing a dispersion comprising solid particles, liquid droplets or gas bubbles dispersed, as an internal phase, in a fluid, as an external phase, by means of a surfactant, as a dispersing agent, said method comprising the steps of
a) providing a surfactant precursor as defined above,
b) activating said surfactant precursor to a surfactant,
c) dispersing a mixture of said internal phase, said external phase and said surfactant.

Also further, the objects of the present invention are met by a method for preparing a dispersion comprising solid particles, liquid droplets or gas bubbles dispersed, as an internal phase, in a fluid, as an external phase, by means of a surfactant as a dispersing agent, said method comprising the steps of
a) providing a mixture of said internal phase, said external phase and a surfactant precursor as defined above,
b) activating said surfactant precursor to a surfactant,
c) dispersing said mixture.

Further, the objects of the present invention are accomplished by a method for breaking a dispersion comprising solid particles, liquid droplets or gas bubbles dispersed, as an internal phase, in a fluid, as an external phase, by means of a surfactant as a dispersing agent, said method comprising the steps of
a) providing a surfactant precursor as defined above,
b) activating said surfactant precursor to a surfactant,
c) dispersing a mixture of said internal phase, said external phase and said surfactant,
d) providing said dispersion at a temperature where it is substantially stable, and
e) setting the temperature of said dispersion, so as to achieve a desired decomposition rate of the surfactant.

Also further, the objects of the present invention are accomplished by a method for breaking a dispersion comprising solid particles, liquid droplets or gas bubbles dispersed, as an internal phase, in a fluid, as an external phase, by means of a surfactant as a dispersing agent, said method comprising the steps of
a) providing a mixture of said internal phase, said external phase and a surfactant precursor as defined above,
b) activating said surfactant precursor to a surfactant,
c) dispersing said mixture,
d) providing said dispersion at a temperature where it is substantially stable, and
e) setting the temperature of said dispersion, so as to achieve a desired decomposition rate of the surfactant.

Such provision as in step a) allows for the surface activity in a composition comprising the surfactant precursor to be switched on at a desirable occasion, thereby changing the properties of the composition.

As described above, the surfactant obtained upon activation of a surfactant precursor of the invention is susceptible to an increased rate of decomposition and inactivation by increasing temperature. This property allows for controlled breaking of a dispersion at a desirable moment and/or at a desirable rate. Hence, in any suitable application, the properties of an initially dispersed composition may be changed as desirable.

The skilled man is able to routinely examine the required temperature for a desired decomposition rate. The temperature is dependent on the surfactant, the external phase and the internal phase components, as well as on pH (see above).

The dispersion is preferably provided at a temperature in the range from about 0 to about 40 °C, preferably from about 10 to about 30 °C, at which the dispersion is virtually stable (i.e. stable for e.g. hours or days).

By raising the temperature to a range from about 40 to about 100 °C, preferably from about 60 to about 95 °C, the dispersion is broken faster due to increased rate of decomposition and deactivation of the surfactant (i.e. decomposition within e.g. minutes or hours). The rate of decomposition, as manifested by delayed dispersion breaking and/or the breaking rate, can thus be effectively controlled by temperature at substantially constant pH conditions. The rate of decomposition generally increases with increasing temperature.

An additional aspect of the present invention is a microemulsion comprising, as surfactant, a compound obtained by activation of a surfactant precursor as defined above with reference to formula I. As is known to the person of skill in the art, a microemulsion is a macroscopically homogeneous mixture of oil, water and surfactant, which on the microscopic level consists of individual domains of oil and water, separated by a monolayer of the surfactant amphiphile. See e.g. Jönsson B et al., "Surfactants and Polymers in Aqueous Solution", John Wiley & Sons Ltd (1998), chapter 18. In analogy to what is explained above, the properties of the surfactant obtained upon activation of the surfactant precursor enables controlled breaking of the microemulsion. The detailed description above regarding the behaviour of a dispersion comprising the compound obtained upon activation of the surfactant precursor as surfactant is equally applicable to the inventive microemulsion, for example in terms of methods for breaking the microemulsion.

Furthermore, the objects of the present invention are attained by the provision of a method for reducing the surface tension between a liquid and another phase, comprising adding to said liquid a surfactant precursor as defined above, and activating said surfactant precursor to a surfactant. Particular variants and options for the surfactant precursor used in the inventive method for reducing surface tension are as discussed above.

The present invention also encompasses several methods and applications, in which the beneficial advantages of the surfactant properties of the compounds obtained by activation of a surfactant precursor as described above are exploited. In the following discussion of such methods and applications, the person of skill in the art can determine which particular surfactant precursor of the class described herein should be used and in what proportions, in order to obtain a suitable surfactant upon activation of said compound, taking into account for example the properties of HLB and Krafft temperature discussed above, as well as the particular chemical nature of the hydrophobic groups.

One application aspect of the present invention relates to use of a compound as described above as a surfactant precursor in the preparation of a lubrication composition. In this context, a surfactant obtained by activation of a surfactant precursor according to the invention is used to form an oil-in-water emulsion of an oily component. Optionally, an extreme pressure (EP) additive and/or a corrosion inhibitor is/are added, in order to enhance performance at high pressures and corrosion resistant properties, respectively. The emulsion may then be used as lubricant. The beneficial properties of the surfactant will enable breaking of the emulsion at a certain elevated temperature, following which the different components are easily separable. This provides for re-use or destruction of the oily phase.

Another application aspect of the present invention relates to use of a surfactant precursor as described above in the preparation of a degreasing composition. To prepare such a degreasing composition, a surfactant precursor as described herein is suitably mixed with a hydrotrope, such as commercially available Ampholac YJH40, a complexing agent, such as Na₃NTA or tripolyphosphate, and an alkaline salt for use as corrosion inhibitor and anti-redeposition agent, such as Na-metasilicate in an aqueous solution. Optionally, an auxiliary surface active compound, such as Berol 266 (Akzo Nobel), is also added. Vigorous stirring of the mixture and alkalinity provides for hydrolytic conditions, under which the surfactant precursor is activated. Following this, white spirit is dissolved or emulsified into the mixture in an amount less than that causing separation of phases. The result is a formulation suitable for use as a degreasing formulation, e.g. in automatic car washing equipment. As in other applications of the inventive concept, the surfactant obtained by activation of the surfactant precursor degrades over time or with raised temperature, which facilitates the separation and further processing of the different components of the formulation after use.

Yet another application aspect of the present invention relates to use of a surfactant precursor as described above in the preparation of a cleaning solution, which has a simpler composition than that described immediately above. A surfactant precursor as described herein may be admixed in suitable proportions with an alkaline component, for example solid potassium hydroxide dissolved in ethanol. The result, after hydrolysis, is an ethanolic concentrate of an activated surfactant precursor according to the invention. The concentrate may then be diluted at the user's discretion, and used for cleaning/degreasing in household and industrial settings.

A further application aspect of the present invention relates to use of a surfactant precursor as described above in the preparation of an alkaline detergent composition, which is useful for example in automatic dishwashing equipment. In preparing such a composition, an aqueous, strongly alkaline (typically pH 11-14) pre-mixture is made of a complexing agent, such as Na₃NTA or tripolyphosphate, and an alkaline salt for use as corrosion inhibitor and anti-redeposition agent, such as Na-metasilicate. Optionally, an auxiliary surface active compound, such as Berol 266 (Akzo Nobel), is also added. To this strongly alkaline pre-mixture, a surfactant precursor as described herein is added under vigorous stirring, creating an emulsion which may be used in dishwashing equipment. During the initial period of use, the surfactant precursor will be activated. With a careful choice of surfactant and proportions of the composition components, the activated surfactant will be degraded during the course of a typical washing program, so that only small amounts, if any, of the intact composition will remain at the end of the program.

Yet a further application aspect of the present invention concerns use of a surfactant precursor as described above in the preparation of a laundry detergent composition. Components of this formulation may be exchanged by the skilled person to provide alternative compositions, taking into account the functional significance of each component. A laundry detergent comprising a surfactant obtained by activation of a surfactant precursor in accordance with the present invention, i.e. a thermolabile (soap-like) surfactant instead of an ordinary soap, may display an enhanced stain removal efficiency due to the fact that the surfactant affinity difference (SAD) / critical packing parameter (CPP) / spontaneus curvature of the surfactant layer between the soil and the aqueous phase will change during the wash cycle as the surfactant breaks down. It is well known that there is an optimal SAD/CPP for optimal removal of each type of oily soil. Using a dynamic detergent as described herein, several different stains can be optimally removed during the same wash cycle, at a constant washing temperature. That this effect can be obtained with a surfactant that breaks down to environmentally friendly products is naturally an additional benefit.

Yet a further application aspect of the present invention concerns use of a surfactant precursor as described above in the preparation of an emulsion of an active substance intended to be applied to agricultural crops. In this context, such an active substance, which for example may be a pesticide, a weed-control agent or other compound intended for protection of the crops, is admixed with activated surfactant precursor and for example propylene glycol to prepare a concentrated stock. The active substance may be provided in any liquid or solid form. When used in the field, the concentrated stock is suitably diluted and used for crop protection. Again, the surfactant obtained by activation of the surfactant precursor according to the invention is beneficial, in that its degradation products are not harmful to the environment with a suitable choice of hydrophobic groups.

Another application aspect of the present invention concerns use of a surfactant precursor as described above in the preparation of an alkyd emulsion for paint formulations. Components of this formulation may be exchanged by the skilled person to provide alternative compositions, taking into account the functional significance of each component. The obtained alkyd emulsion can be used in the formulation of e.g. high gloss paints for outdoor use. Alkyd emulsions stabilised by a decomposable surfactant obtained by activation of a surfactant precursor according to the invention may allow formulation of paints with enhanced water resistance, due to the transformation of the water soluble surfactant (after application of paint) into an oil-like hydrophobic residue. The hydrophobic nature of the decomposition product may also render the painted surface more water repellent/hydrophobic.

Yet another application aspect of the present invention concerns use of a surfactant precursor as described above in the preparation of an ink composition. Components of this formulation may be exchanged by the skilled person to provide alternative compositions, taking into account the functional significance of each component. The obtained ink is useful for printing, e.g. in a flexographic printing process. After printing, the surfactant will break down to harmless non-surfactant products with time or through application of heat. Apart from the obvious environmental benefit, adhesion of any subsequent coatings on top of the printed area may be enhanced due to the breakdown of the surfactant. Furthermore, the dried ink may exhibit a better resistance towards water or waterborne chemicals, as the decomposition products typically have a much lower solubility in water, compared to the surfactant.

Yet another application aspect of the present invention concerns use of a surfactant precursor as described above in the preparation of a coating composition. Components of this formulation may be exchanged by the skilled person to provide alternative compositions, taking into account the functional significance of each component. The coating formulation obtained may be used for paper/board coating. The decomposition of the surfactant in the formulation (in the drying step right after application of the coating, later upon storage or by heat treatment for the explicit purpose of surfactant decomposition) may provide benefits such as better printing properties, better adhesion (e.g. towards a polyethylene coating applied in a subsequent step) and enhanced friction properties. In addition, the non-toxicity of the surfactant degradation products (suitable hydrophobic groups and carbonate/hydrogen carbonate) is beneficial for paper and/or board intended to be in contact with foodstuffs.

### Examples

### Example 1a. Transformation of liquid AKD into a decomposable surfactant through hydrolysis

Ten parts by weight of liquid AKD (the clear liquid fraction obtained after centrifugation of the product Raisares A20 (Ciba Specialty Chemicals Oy, Finland)) were mixed with 61 parts of a saturated (ca 0.5 M) solution of potassium hydroxide dissolved in dry ethanol containing maximum 0.3 vol% water.

The obtained solution was left to stand for 215 minutes at room temperature to allow hydrolysis of the AKD to take place. The obtained solution thereafter contained the active decomposable surfactant. The surfactant can be used as it is, as long as ethanol is not a problem (e.g. flashpoint issues, etc.) in the actual application.

The ethanol was removed by evaporation at room temperature, by letting a flow of compressed air pass over the liquid to speed up the evaporation. The remaining semi-solid was thereafter dissolved/dispersed in water, by adding 797 parts by weight of water followed by mixing. A cloudy, foaming and soap-like solution was thereby obtained.

### Example 1b. Transformation of solid AKD into a decomposable surfactant through hydrolysis

Ten parts by weight of solid AKD (the solid with melting point 62 °C, R¹ & R² either hexadecyl (92% of mixture) or tetradecyl (6% of mixture), provided by EKA Chemicals AB, Sweden) were mixed with 156 parts of a saturated (ca 0.5 M) solution of potassium hydroxide dissolved in dry ethanol containing maximum 0.3 vol% water.

The obtained mixture was left to stand under constant stirring for 110 minutes at room temperature to allow hydrolysis of the AKD to take place. Full dissolution of the solid AKD may not have taken place, and the liquid became cloudy as a result of formation of the soap-like surfactant.

After 110 minutes of hydrolysis, the liquid was decanted to remove any remaining sediment of un-reacted AKD.

The obtained cloudy liquid now contained the active decomposable surfactant. The liquid containing the surfactant can be used as it is, as long as ethanol is not a problem (e.g. flashpoint issues, etc.) in the actual application.

The ethanol was removed by evaporation at room temperature, by letting a flow of compressed air pass over the liquid to speed up the evaporation. The remaining semi-solid was thereafter dissolved/dispersed in water, by adding 310 parts by weight of water followed by mixing. A cloudy, foaming and soap-like solution was thereby obtained.

### Example 1c. Transformation of liquid AKD into a decomposable surfactant through hydrolysis in aqueous solution

50 µl of the clear liquid fraction obtained after centrifugation of the product Raisares A20 (Ciba Specialty Chemicals Oy, Finland) (cf. Example 1a) was mixed with 1.5 ml of a 0.5 M NaOH solution and thoroughly shaken. The AKD was not dissolved or dispersed, but floating white flakes were seen on top of a clear solution. The sample was then heated at 65 °C for 56 minutes and shaken from time to time. After this treatment, the sample did not give a stable foam upon shaking at 65 °C. Further heat treatment at 93.5 °C for 275 minutes (with periodic shaking of the sample) resulted in a solution giving a stable foam of 8 mm height upon shaking at 93.5 °C. Furthermore, the flakes were now replaced by a seemingly stable, cloudy dipersion which is the typical appearance of the liquid formed after successful hydrolysis of AKD according to e.g. Example 1a.

### Example 2. Verification of the expected acid-base behaviour of the obtained surfactants

Addition of drop-wise amounts of 1 M HCl to samples of the ethanol-free aqueous surfactant mixtures prepared in Example 1a and 1b ultimately resulted in a precipitation of the beta-keto acid, which was insoluble in water. The pH-value at which the precipitation occurs depends on, and reflects, the pKa of the surfactant in question.

For the surfactant mixture prepared from liquid AKD according to Example 1a (the Ciba product), precipitation occurred at a pH value of between 6.85 and 4.50.

For the corresponding mixture prepared from solid AKD (Example 1b, the product from EKA Chemicals) precipitation of solid flakes was initiated at a pH value between 12 and 9.

The above observed behaviour of the surfactant mixtures show that the hydrolysis products are indeed surfactants of the described type.

### Example 3. Verification of the decomposing character of the surfactant - decrease in foamability with time

An ethanol-free surfactant solution prepared e.g. as described in Example 1b was diluted with deionised water to a concentration just above the concentration where foaming at 95 °C would start to decrease if the solution were further diluted (measured as the foam height obtained in a test tube 5 seconds after vigorous shaking of the hot sample).

The magnitude of dilution needed depends on the exact chemical composition of the AKD precursor, the critical micelle concentration (CMC) of the actual surfactant, the hydrolysis time, and the amount of un-reacted AKD during hydrolysis. A relatively pure AKD precursor, containing only low levels of non-decomposable surface active components (e.g. fatty acids, etc.), was used in order to demonstrate the surfactant decomposition through studies of decrease in foamability.

When a satisfactory concentration, just above the critical concentration below which foaming cannot occur, had been established, and it had been tested that this concentration would give a voluminous and stable foam after a minute or two of heating at 95 °C, a fresh (non-heated) sample of this concentration was prepared and heated to 95 °C in a heating bath. Foam height 5 seconds after shaking was then monitored as a function of time.

Typical results can be found in Table 2. Note that for a more concentrated solution the decrease in foamability will occur after longer heat treatment times, as the time needed for the surfactant concentration to be lowered below the critical level, where foaming is not possible, is prolonged.

Table 2 shows the typical behaviour of samples prepared as described above. The results in the first two columns of the table (Sample A and Sample B) were obtained using ethanol-free surfactant solutions prepared according to Example 1b (using the EKA Chemicals solid AKD product). The solutions were first diluted as described in Example 1b, and then further diluted as indicated in the table. Note that the indicated concentrations are nominal values; the real concentration were clearly lower, as substantial amounts of the solid AKD was left un-reacted after the hydrolysis in Example 1b.

The results presented in the rightmost column of the table (Sample C) were obtained with a solution prepared according to Example 1c, which had been diluted 265 times. The fact that a decrease in foaminess with time was obtained also for this sample, shows that hydrolysis of liquid AKD directly in an alkaline aqueous medium is indeed possible at elevated temperatures, and results in a decomposable surfactant.

**Table 2.**

| Time (min) | Foam height at 95 °C 5 seconds after shaking(mm) | Foam height at 95 °C 5 seconds after shaking(mm) | Foam height at 93-95 °C 5 seconds after shaking(mm) |
|---|---|---|---|
| | SAMPLE A, diluted 80.7 times, pH = 10 | SAMPLE B, diluted 20.7 times, pH=11 | SAMPLE C, diluted 265 times |
| 2 | 10¹ | | |
| 5 | 9 | | 6 |
| 8 | 4² | | |
| 10 | 3² | | |
| 14 | | | 4 |
| 16 | 0 | | 4 |
| 105 | | | 0 |
| 115 | | >10 | |
| 131 | | 13 | |
| 165 | | 6 | |
| 169 | | 7 | |
| 201 | | 5 | |
| 229 | 0 | | |
| 236 | | 3² | |
| 247 | | 0 | |

| | | | |
|---|---|---|---|
| 1) Stable foam 2) A ring of foam around the rim of the test tube only | | | |

### Example 4. Verification of thermolability of the AKD-derived surfactants through study of emulsion-breaking at elevated temperature.

Two ethanol-free surfactant solutions/dispersions were prepared according to Examples 1a and 1b, respectively. The solutions were further diluted so as to arrive at low concentrations of surfactant, see Table 3 below.

Alternatively, in parallel to Example 3, the surfactant concentration could be experimentally adjusted so as to be just above the level needed for satisfactory emulsification.

Emulsion samples (A, B, C and D, see Table 3) were then prepared according to the following steps, using the two diluted ethanol-free surfactant solutions referred to above.

1.2 grams of hexadecane (coloured with an oil soluble pigment) were mixed with 6.4 grams of the surfactant solution. Thereafter, an emulsion was prepared from the mixture using an Ultrathurrax high shear mixing equipment, operated at 20 000 rpm for 2 minutes.

The pH of the emulsions was then adjusted to the desired pH (see Table 3) by means of small additions of 1 M NaOH and/or 1 M HCl.

The emulsion samples were then heated to 95 °C (samples A, C and D) and to 65 °C (Sample B).

After heating of the emulsions, they were all fully or partially broken (e.g. separated into an oil phase and and aqueous phase) after a shorter or longer period of heat treatment (minutes to hours). A coloured oil phase was thereby found floating on top of the aqueous phase. The time where partial or full emulsion breaking was observed can be found in Table 3.

The heat treatment was continued for a significant time after separation of the emulsions (see Table 3), and then the samples were brought to room temperature followed by re-emulsification of the samples using the same protocol as used to prepare the emulsions (Ultrathurrax mixer at 20 000 rpm for 2 minutes, see above)

It was found that it was not possible to fully re-emulsify the samples (not possible to get the oil stabilised as small droplets in the emulsion), but rather that all, or a large portion of, the oil (50-100 %) separated and formed a separate oil phase immediately after the re-emulsification attempt. This shows that the surfactant that once stabilised the emulsion had broken down to non-surface active products during the heat treatment, and that the formed products were not capable of stabilising the emulsion.

The experimental data for samples A, B, C and D are summarised in Table 3.

**Table 3.**

| Sample label | Precursor | Emulsion fully broken after (min) | Nominal surfactant concentration in aqeuous phase (calc. as wt% AKD) | pH | Heat treatment time (min) | T (°C) | Estimated amount of oil separating directly after re-emulsification |
|---|---|---|---|---|---|---|---|
| A | Liquid AKD (Ciba) | 189 | 0.035 | 12 | 395 | 95 | 100 % |
| B | Liquid AKD (Ciba) | 189 (not fully broken) | 0.035 | 12 | 395 | 65 | 50 % |
| C | Solid AKD (EKA Chemicals) | 447 | 0.15¹ | 11 | 588 | 95 | 100 % |
| D | Solid AKD (EKA Chemicals) | 219 | 0.040¹ | 10 | 357 | 95 | 100 % |

| | | | | | | | |
|---|---|---|---|---|---|---|---|
| 1) The actual concentration was lower due to remaining non hydrolysed solid AKD | | | | | | | |

### Example 5. Investigation of the type of emulsions obtained

Emulsions were prepared using the ethanol-free solution prepared in Example 1b and the protocol described in Example 4 (coloured hexadecane, Ultrathurrax etc). Three samples of the emulsion were then adjusted with 1 M NaOH/1 M HCl to pH 8, 10 and 12, respectively and stored at room temperature for more than 10 hours. The emulsions were thereafter re-emulsified, and checked for type of emulsion (oil-in-water or water-in-oil).

It was verified that the obtained emulsions were of the expected oil-in-water type, through checking the possibility to disperse a drop of the emulsion in i) water and ii) hexadecane. An oil-in-water emulsion will only be dispersible in water, and not in hexadecane (oil). The emulsions prepared above were clearly found to be of the oil-in-water type.

It shall be noted that all three samples above gave stable emulsions that were stable also four days after the above re-emulsification (stored at room temperature). Only so-called creaming of the emulsions was seen, meaning that a gravitationally driven concentration of the emulsified oil droplets in the top of the test tube had taken place. No free separated oil could be seen before or after a gentle homogenisation of the test tubes, which led to re-dilution of the concentrated creamed emulsion present in the top of the test tube.

## Claims

1. Use, as a surfactant precursor, of a compound having the general formula I 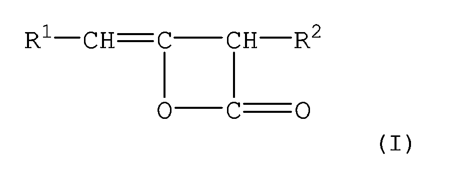 wherein at least one of R¹ and R² is a hydrophobic moiety.

2. Use of a compound as defined in claim 1 as a precursor for a surfactant having the general formula II 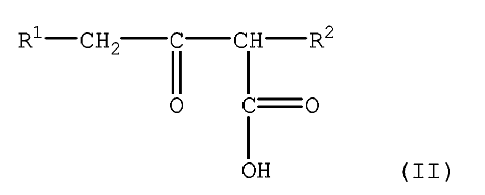 wherein at least one of R¹ and R² is a hydrophobic moiety, or a salt of said surfactant.

3. Use according to claim 1 or 2, wherein R¹ is a hydrophobic moiety.

4. Use according to claim 1, 2 or 3, wherein R² is a hydrophobic moiety.

5. Use according to any one of claims 1-4, wherein R¹ and R² both are hydrophobic moieties of the same type.

6. Use according to any one of the preceding claims, wherein the at least one hydrophobic moiety is independently selected from a straight-chain, branched-chain or cyclic, saturated or unsaturated, optionally substituted, aliphatic group; an optionally substituted aromatic group; an optionally substituted hydrophobic polyoxyalkylene group; an optionally substituted perfluoroalkyl group; an optionally substituted polysiloxane group; a lignin or rosin derivative; and any combination thereof.

7. Use according to claim 6, wherein the at least one hydrophobic moiety is independently selected from a straight-chain, branched-chain or cyclic, saturated or unsaturated, optionally substituted, aliphatic group; an optionally substituted aromatic group; and a combination thereof.

8. Use according to claim 7, wherein the at least one hydrophobic moiety independently is a straight-chain or branched-chain, saturated or unsaturated, optionally substituted, C₄-C₃₀ alkyl.

9. Use according to claim 8, wherein the at least one hydrophobic moiety independently is a straight-chain or branched-chain, saturated or unsaturated, optionally substituted, C₄-C₂₂ alkyl.

10. Use according to claim 9, wherein the at least one hydrophobic moiety is independently selected from a group consisting of: -(CH₂)₃-CH₃; -(CH₂)₅-CH₃; -(CH₂)₇-CH₃; -(CH₂)₉-CH₃; -(CH₂)₁₁-CH₃; -(CH₂)₁₃-CH₃; -(CH₂)₁₅-CH₃; -(CH₂)₁₇-CH₃; -(CH₂)₁₉-CH₃; -(CH₂)₆-CH=CH-(CH₂)₅-CH₃; - (CH₂)₁₃-CH(CH₃)-CH₃; -(CH₂)₆-CH=CH-(CH₂)₇-CH₃; - (CH₂)₆-CH=CH-CH₂-CH=CH-(CH₂)₄-CH₃; - (CH₂)₆-CH=CH-CH₂-CH=CH-CH₂-CH=CH-CH₂-CH₃; - (CH₂)₆-CH=CH-(CH₂)₉-CH₃; and - (CH₂)₁₀-CH=CH-(CH₂)₇-CH₃.

11. Use according claim 10, wherein the at least one hydrophobic moiety is independently selected from a group consisting of: -(CH₂)₉-CH₃; -(CH₂)₁₁-CH₃; -(CH₂)₁₃-CH₃; -(CH₂)₁₅-CH₃; -(CH₂)₁₉-CH₃; -(CH₂)₁₃-CH(CH₃)-CH₃; - (CH₂)₆-CH=CH-(CH₂)₇-CH₃; -(CH₂) ₆-CH=CH-CH₂-CH=CH-(CH₂)₄-CH₃; - (CH₂) ₆-CH=CH-CH₂-CH=CH-CH₂-CH=CH-CH₂-CH₃; - (CH₂) ₆-CH=CH- (CH₂)₉-CH₃; and - (CH₂)₁₀-CH=CH- (CH₂)₇-CH₃.

12. Use according to claim 11, wherein the at least one hydrophobic moiety is independently selected from - (CH₂)₁₃-CH₃ and - (CH₂)₁₅-CH₃.

13. A dispersion comprising solid particles, liquid droplets or gas bubbles dispersed, as an internal phase, in a fluid, as an external phase, by means of a surfactant, as a dispersing agent, wherein said surfactant is obtained by activation of a surfactant precursor as defined in any one of the preceding claims.

14. A dispersion according to claim 13, which is an aerosol, a colloid, an emulsion, a foam, a gel, a sol or a suspension.

15. A method for preparing a dispersion comprising solid particles, liquid droplets or gas bubbles dispersed, as an internal phase, in a fluid, as an external phase, by means of a surfactant, as a dispersing agent, said method comprising the steps of
a) providing a surfactant precursor as defined in any one of claims 1-12,
b) activating said surfactant precursor to a surfactant,
c) dispersing a mixture of said internal phase, said external phase and said surfactant.

16. A method for preparing a dispersion comprising solid particles, liquid droplets or gas bubbles dispersed, as an internal phase, in a fluid, as an external phase, by means of a surfactant as a dispersing agent, said method comprising the steps of
a) providing a mixture of said internal phase, said external phase and a surfactant precursor as defined in any one of claims 1-12,
b) activating said surfactant precursor to a surfactant,
c) dispersing said mixture.

17. A method for breaking a dispersion comprising solid particles, liquid droplets or gas bubbles dispersed, as an internal phase, in a fluid, as an external phase, by means of a surfactant, as a dispersing agent, said method comprising the steps of
a) providing a surfactant precursor as defined in any one of claims 1-12,
b) activating said surfactant precursor to a surfactant,
c) dispersing a mixture of said internal phase, said external phase and said surfactant,
d) providing said dispersion at a temperature where it is substantially stable, and
e) setting the temperature of said dispersion, so as to achieve a desired decomposition rate of the surfactant.

18. A method for breaking a dispersion comprising solid particles, liquid droplets or gas bubbles dispersed, as an internal phase, in a fluid, as an external phase, by means of a surfactant as a dispersing agent, said method comprising the steps of
a) providing a mixture of said internal phase, said external phase and a surfactant precursor as defined in any one of claims 1-12,
b) activating said surfactant precursor to a surfactant,
c) dispersing said mixture,
d) providing said dispersion at a temperature where it is substantially stable, and
e) setting the temperature of said dispersion, so as to achieve a desired decomposition rate of the surfactant.

19. A method according to any one of claims 17-18, wherein steps a) - d) are performed at a temperature in the range from about 0 to about 40 °C, preferably from about 10 to about 30 °C.

20. A method according to any one of claims 17-19, wherein step e) is performed by raising the temperature to a range from about 40 to about 100 °C, preferably from about 60 to about 95 °C.

21. A method for reducing the surface tension between a liquid and another phase, comprising
a) adding to said liquid a surfactant precursor as defined in any one of claims 1-12 and
b) activating said surfactant precursor to a surfactant.

22. A method according to any one of claims 15-21, wherein said surfactant precursor is activated by hydrolysis.

23. A microemulsion comprising, as a surfactant, a compound obtained by activation of a surfactant precursor as defined in any one of claims 1-12.

24. A method for breaking a microemulsion according to claim 23, said method comprising the steps of
a) providing said microemulsion at a temperature where it is substantially stable; and
b) setting the temperature of said microemulsion, so as to achieve a desired decomposition rate of the surfactant.

25. A method according to claim 24, wherein step a) is performed at a temperature in the range from about 0 to about 40 °C, preferably from about 10 to about 30 °C

26. A method according to claim 24 or 25, wherein step b) is performed by raising the temperature to a range from about 40 to about 100 °C, preferably from about 60 to about 95 °C.

27. Use of a compound as defined in any one of claims 1-12 as a surfactant precursor in the preparation of a lubrication composition.

28. Use of a compound as defined in any one of claims 1-12 as a surfactant precursor in the preparation of a degreasing composition.

29. Use of a compound as defined in any one of claims 1-12 as a surfactant precursor in the preparation of a cleaning solution.

30. Use of a compound as defined in any one of claims 1-12 as a surfactant precursor in the preparation of an alkaline detergent composition.

31. Use of a compound as defined in any one of claims 1-12 as a surfactant precursor in the preparation of a laundry detergent composition.

32. Use of a compound as defined in any one of claims 1-12 as a surfactant precursor in the preparation of an emulsion of an active substance intended to be applied to agricultural crops.

33. Use of a compound as defined in any one of claims 1-12 as a surfactant precursor in the preparation of an alkyd emulsion for paint formulations.

34. Use of a compound as defined in any one of claims 1-12 as a surfactant precursor in the preparation of an ink composition.

35. Use of a compound as defined in any one of claims 1-12 as a surfactant precursor in the preparation of a coating composition.
